# EUROPEAN PATENT APPLICATION

(11) **EP 3 659 552 A1**
(43) Date of publication of application: **03.06.2020**
(21) Application number: 19211409.8
(22) Date of filing: 26.11.2019
(51) Int. Cl.: A61F 2/24

(54) **TRANSCATHETER MITRAL VALVE LEAFLET EXTENSION**

(30) Priority: 28.11.2018 US 201862772188 P
(71) Applicant: St. Jude Medical, Cardiology Division, Inc., St. Paul MN 55117 (US)
(72) Inventor: REIMER, Jay, Saint Paul, MN 55101 (US); MOORE, Brandon, St. Louis Park, MN 55416 (US); GREEN, Chad Joshua, Forest Lake, MN 55025 (US); ASHWORTH, Paul E., Danbury, WI 54830 (US); SAIKRISHNAN, Neelakantan, Plymouth, MN 55446 (US)
(74) Representative: Gill Jennings & Every LLP

(57) **Abstract**

A method of augmenting the length of a native leaflet to improve leaflet coaptation includes delivering a cutting tool to a surgical site adjacent the native leaflet, cutting a slit through the native leaflet, inserting a collapsible and expandable occlusion device into the slit, expanding the occlusion device, engaging a first surface of the first disk with a first surface of the native leaflet, and engaging a second surface of the second disk with a second surface of the native leaflet opposite the first surface. The occlusion device includes a first disk, a second disk, and an intermediate portion coupling the first disk to the second disk.

## Description

### BACKGROUND OF THE INVENTION

The present invention generally relates to heart valve repair and, more particularly, to apparatus and methods for augmenting heart valve leaflets.

Properly functioning heart valves can maintain unidirectional blood flow in the circulatory system by opening and closing, depending on the difference in pressure on opposite sides of the valve. The two atrioventricular valves (mitral and tricuspid valves) are multicuspid valves that prevent backflow from the ventricles into the atria during systole. They are anchored to the wall of the ventricle by chordae tendineae, which prevent the valves from inverting.

The mitral valve is located at the gate between the left atrium and the left ventricle and is made up of two leaflets and a diaphanous incomplete ring around the valve, known as the mitral valve annulus. When the valve opens, blood flows into the left ventricle. After the left ventricle fills with blood and contracts, the two leaflets of the mitral valve are pushed upwards and close, preventing blood from flowing back into the left atrium and the lungs.

Mitral valve tenting is a type of valve disease in which the mitral valve leaflets tent (i.e., a portion of the affected leaflet is bulged or raised), preventing the leaflets from properly coapting. Accordingly, as the ventricle contracts, blood is allowed to return to the left atrium and the lungs. This phenomenon is known as mitral regurgitation.

One cause of mitral valve tenting is ventricular enlargement due to pathophysiological remodeling, which may occur, for example, following a heart attack. Another cause of mitral valve tenting is shortened and/or inelastic chordae tendineae. The shortened and/or inflexible chordae tendineae prevent the leaflets to which they are attached from properly coapting and result in ischemic mitral regurgitation. Still yet another cause of mitral valve tenting is shortened and/or inelastic mitral valve leaflets that are incapable of properly coapting due to their shortened length. It has been discovered that as many as half of all myocardial infarction patients develop ischemic mitral regurgitation.

Untreated mitral regurgitation may lead to congestive heart failure and pulmonary hypertension. For this reason, mitral regurgitation is often treated using annuloplasty rings, relocating papillary muscles, cutting chordae tendineae, or replacing the entire mitral valve.

Despite the various improvements that have been made to these treatment devices and methods, various shortcomings remain. For example, conventional treatment methods typically require invasive open heart surgery, which often requires an extended recovery period.

There therefore is a need for improvements to the devices and methods for repairing tented mitral valve leaflets using minimally invasive techniques. Among other advantages, the present disclosure addresses these needs.

### BRIEF SUMMARY OF THE INVENTION

In accordance with a first aspect of the present disclosure, a method for augmenting the length of a native leaflet within a diseased atrioventricular valve is provided. Among other advantages, the method allows the diseased valve to be repaired using minimally invasive techniques.

One embodiment of the method includes delivering a cutting tool to a surgical site adjacent the native leaflet; cutting a slit through the native leaflet; inserting a collapsible and expandable occlusion device into the slit, the occlusion device having a first disk, a second disk, and an intermediate portion coupling the first disk to the second disk; expanding the occlusion device; engaging a first portion of the first disk with a first surface of the native leaflet; and engaging a first portion of the second disk with a second surface of the native leaflet opposite the first surface.

Another aspect of the present disclosure provides an apparatus for augmenting the length of a native leaflet. The apparatus includes a delivery device having a lumen; and a collapsible and expandable plug having a first disk, a second disk, and an intermediate portion coupling the first to the second disk. The first disk has a concave surface for engaging the native leaflet and the second disk includes a convex surface for engaging the native leaflet.

### BRIEF DESCRIPTION OF THE DRAWINGS

Various embodiments of the present disclosure are described herein with reference to the following drawings in which:
FIG. 1 is a schematic cutaway view of a human heart;
FIG. 2A is a schematic representation of a native mitral valve and associated structures during normal operation;
FIG. 2B is a schematic cross-sectional view taken along a longitudinal axis of the native mitral valve and illustrating an exemplary tented mitral valve having a shortened native chordae tendineae;
FIG. 3 is a schematic front view of an augmentation apparatus according to an embodiment of the present disclosure;
FIG. 4 is a front elevational view of an occlusion device according to an embodiment of the present disclosure; and
FIGS. 5A-6D are schematic views showing the use of the augmentation apparatus of FIG. 3 to insert the occlusion device of FIG. 4 into a slit in the native leaflet to augment the length of the native leaflet.

### DETAILED DESCRIPTION

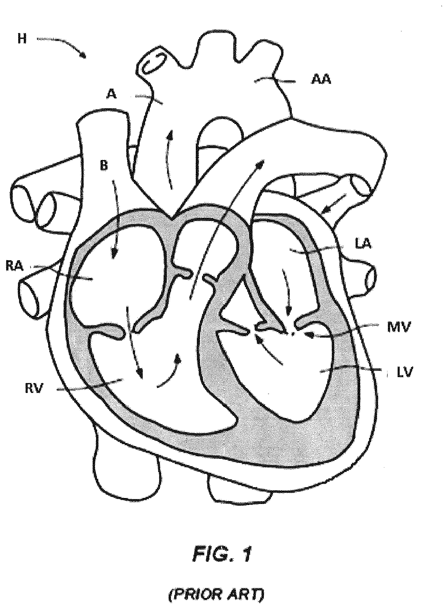

FIG. 1 is a schematic representation of a human heart H. The human heart includes two atria and two ventricles: a right atrium RA and a left atrium LA, and a right ventricle RV and a left ventricle LV. Blood flows through the heart H in the direction shown by arrows "B". As illustrated in FIG. 1, the heart H further includes an aorta A and an aortic arch AA. Disposed between the left atrium LA and the left ventricle LV is the mitral valve MV. The mitral valve MV, also known as the bicuspid valve or left atrioventricular valve, is a bi-leaflet valve that opens as a result of increased pressure in the left atrium, relative to the left ventricle, as the left atrium fills with blood.

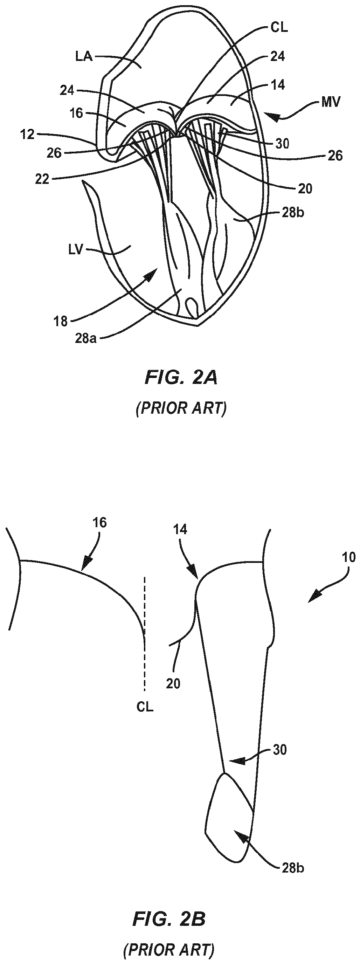

A typical mitral valve MV, an example of which is shown in FIG. 2A, includes an annulus 12, a posterior leaflet 14, an anterior leaflet 16, and sub-valvular structure 18. Annulus 12 is a dense ring of fibrous tissue which lies at the juncture between the left atrium and the left ventricle. Posterior leaflet 14 and anterior leaflet 16 are attached to annulus 12 and extend toward the valve orifice. The portions of posterior and anterior leaflets 14, 16 that extend toward the valve orifice are known as free edges 20, 22, respectively.

Each of posterior and anterior leaflets 14, 16 has an upper portion 24 that, when the leaflets are closed, extends from annulus 12 to coaptation line CL in a direction that is generally perpendicular to the direction of blood flow through the valve, and a lower portion 26 that, when the leaflets are closed, extends downward from the coaptation line to the free edge of the leaflet in a direction that is generally parallel to the direction of blood flow through the valve. Posterior leaflet 14 and anterior leaflet 16 both have three scalloped portions.

Sub-valvular structure 18 includes two muscular projections that protrude from an inner wall of the left ventricle LV, known as papillary muscles 28a, 28b, and numerous chordae tendineae 30, thin fibrous bundles that emanate from the papillary muscles and attach to a bottom surface of the valve leaflets near the leaflet root.

During atrial systole, blood flows down the pressure gradient from the left atrium LA to the left ventricle LV. When the left ventricle LV contracts during ventricular systole, the increased blood pressure in the left ventricle LV causes the mitral valve leaflets to coapt, preventing the backflow of blood into the left atrium LA. Since the blood pressure in left atrium LA is much lower than the blood pressure in left ventricle LV, posterior and anterior leaflets 14, 16 attempt to evert to the low pressure regions. Chordae tendineae 30 prevent the eversion by becoming tense, thus pulling posterior leaflet 14 and anterior leaflet 16 and holding them in a coapting or closed position.

FIG. 2B is a schematic representation of a diseased mitral valve 10 during ventricular systole. For clarity purposes, a single shortened or inflexible chordae tendineae 30 is depicted. Diseased chordae tendineae 30 is fully extended such that the free edge 20 of posterior leaflet 14 is prevented from extending to coaptation line CL and coapting with anterior leaflet 16. For illustrative purposes, anterior leaflet 16 is depicted in a properly closed position (i.e., adjacent coaptation line CL), although it is recognized that any of the chordae tendineae 30 could shorten or lose flexibility, preventing either or both of posterior leaflet 14 and anterior leaflet 16 from extending to coaptation line CL.

The devices and methods described herein are adapted to repair diseased posterior or anterior leaflets 14, 16, to compensate for diseased chordae tendineae 30, and to restore proper function to the native valve. Instead of completely replacing the native valve, the device augments the diseased leaflets and restores proper coaptation between the leaflets. While the devices and methods are described herein in connection with the repair of the mitral valve, it will be appreciated that these concepts may be equally applicable to the repair of the tricuspid valve.

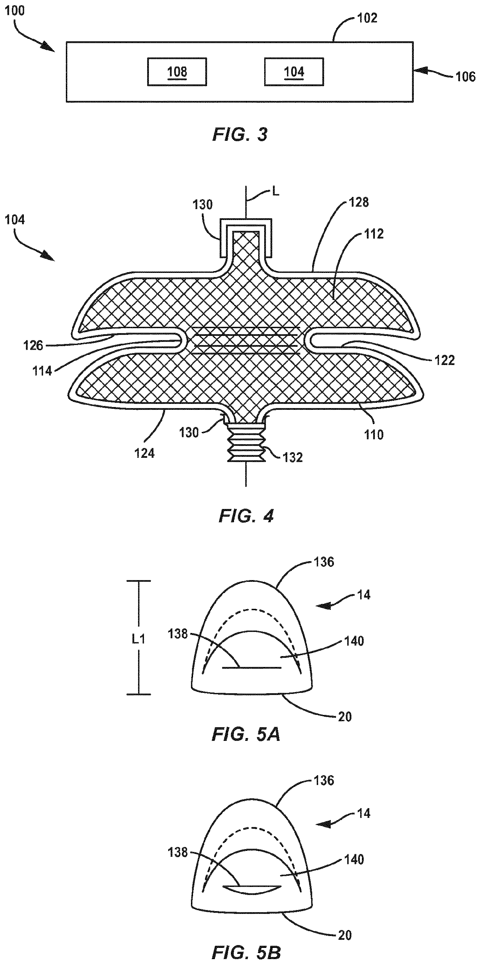

An exemplary augmentation apparatus 100, shown schematically in FIG. 3, includes a delivery device 102 and an occlusion device 104. Delivery device 102 may be a flexible, elongated catheter that extends in a longitudinal direction and that has a lumen 106 for housing occlusion device 104. Delivery device 102 may be delivered to a surgical site located adjacent a diseased leaflet using a trans femoral, transapical, transseptal or other approach known in the art.

Augmentation apparatus 100 may optionally include a cutting device 108 at least partially disposed within the lumen 106 of delivery device 102 for cutting a slit in the diseased mitral valve leaflet. Cutting device 108 may be a mechanical device, for example, a blade, a saw, or scissors. Cutting device 108 may alternatively be a laser or any other device capable of cutting a slit through a native leaflet.

Referring to FIG. 4, occlusion device 104 is a collapsible and expandable plug having a first disk 110, a second disk 112, and an intermediate portion 114 coupling the first disk to the second disks. First disk 110 may sometimes be referred to herein as the proximal disk since, as described below, it is closest to the user during delivery and implantation into a patient. Similarly, disk 112 may sometimes be referred to herein as the distal disk since it is farthest from the user during delivery and implantation. The occlusion device or plug 104 may be formed from a network of braided wires or mesh. Preferably, the braided wire or mesh comprises a biocompatible material that is capable of self-expansion, for example, a shape memory alloy such as nitinol or a self-expanding and bioresorbable material that promotes ingrowth such as polylactide. The nitinol or polylactide mesh or braid may be configured such that plug 104 is substantially dumbbell shaped. In some embodiments, plug 104 may include a plurality of braided layers, each of which may have a dumbbell shape. For example, an outer braided layer may surround an intermediate braided layer which may surround an innermost braided layer. The innermost braided layer may be formed of a higher density braid than the intermediate braided layer which, in turn, may be formed of a higher density braid than the outer layer. As used herein, braids that house a higher density are those that have a greater amount of solid material and a lesser amount of voids per unit of area. As a result of the aforementioned layered structure, plug 104 may have a relatively dense core that is capable of substantially preventing blood flow therethrough without unduly prohibiting the outermost layer from crimping to the collapsed configuration.

In order to improve occlusion and prevent blood flow through plug 104 (and hence through the slit in the native valve leaflet), in some embodiments, the outer structure of plug 104 may be covered with one or more layers of an impervious or relatively impervious material. Such material may be a fabric, a suitable biological material, such as bovine or porcine pericardium, or a polymer, such as PTFE, urethane and the like or a combination of these materials. Alternatively, when plug 104 includes multiple braided layers as described above, one or more layers of these materials may be provided between one or more of the braided layers. Plug 104 may additionally include bioactive molecules to promote tissue ingrowth of the native leaflet such that a new layer of endothelial cells may form over occlusion device 104 and reduce the possibility of bacterial endocarditis or the formation of thromboembolisms.

In the collapsed configuration, occlusion device 104 may be radially crimped (e.g., toward a longitudinal axis L of the plug) to a diameter that allows it to be inserted into the lumen 106 of delivery device 102 for delivery to the surgical site, and then through a slit formed in the diseased mitral valve leaflet. In the expanded configuration, proximal disk 110, distal disk 112 and intermediate portion 114 may have any peripheral shape, for example, circular, elliptical, oval, rectangular, hexagonal or octagonal. However, the expanded cross-settings of disks 110 and 112 in at least one direction orthogonal to longitudinal axis L is greater than the expanded cross-section of the intermediate portion 114 in that direction. Such configuration prevents plug 104 from being dislodged from the slit after it has been inserted therein and expanded.

Proximal disk 110 has an inner surface 122 for engaging one surface of the native leaflet when assembled thereto and an outer surface 124 opposite the inner surface. The inner surface 122 of proximal disk 110 may be convex relative to a plane extending between disks 110 and 112 and orthogonal to the longitudinal axis L of plug 104 to substantially correspond to the morphology of the native leaflet. Similarly, distal disk 112 has an inner surface 126 for engaging the opposite surface of the leaflet when assembled thereto and an outer surface 128 opposite the inner surface. The inner surface 126 of distal disk 112 may be concave relative to a plane extending between disks 110 and 112 and orthogonal to the longitudinal axis L of plug 104 to substantially correspond to the morphology of the native leaflet. In this manner, a substantial portion of the inner surfaces 122, 126 of plug 104 may engage opposing surfaces of the native leaflet and create a superior seal for preventing blood flow through the leaflet slit. This configuration also preserves the natural flapping motion of the native leaflets.

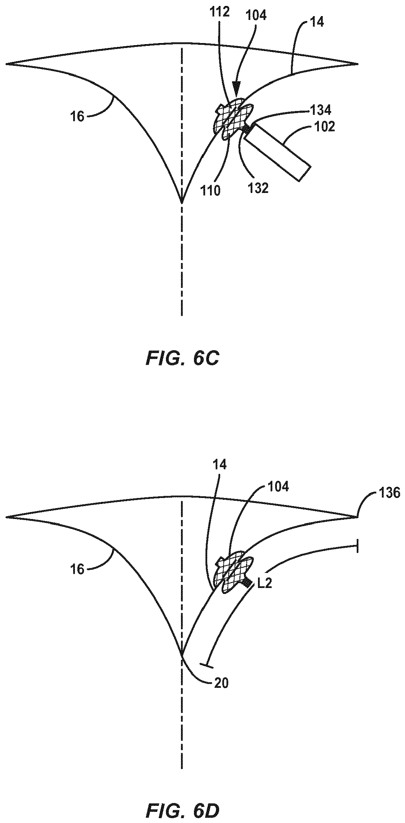

The ends of the braided wires or mesh of plug 104 may be welded or held together via a clamp 130 to prevent the material from fraying. Clamp 130 may be substantially cylindrical in shape and define a recess (not shown) in which the ends of braided wires or mesh are grouped together and secured. Clamp 130 may also include external or internal threads 132 adapted to engage a deployment device 134 (shown in FIG. 6C) having corresponding threads.

As was previously mentioned, diseased mitral valve leaflets may shorten, lose flexibility, or be restrained by inflexible chordae tendineae, thereby preventing the free edge 20 of posterior leaflet 14 from coapting with the free edge 22 of anterior leaflet 16 along coaptation line CL. Referring to FIG. 5A, the distance between the leaflet's base 136 (e.g., the portion of the leaflet that extends from annulus 12) and the free edge 20 of leaflet 14 before the augmentation procedure has been performed is herein referred to as the native length L1.

Augmentation device 100 may be used to augment or lengthen one or more of the leaflets to a length that restores proper mitral valve function. This distance is referred to herein as the augmented length L2 (shown in FIG. 6D) and is defined as the distance between the base 136 of leaflet 14 and the free edge 20 of the leaflet when proper mitral valve function has been restored.

The use of device 100 to augment posterior leaflet 14 will now be described with reference to FIGS. 5A-6D. It will be appreciated, however, that either posterior leaflet 14 or anterior leaflet 16, or both the posterior and anterior leaflets may be augmented as described below.

After catheter 102 has been delivered to the surgical site adjacent posterior leaflet 14, a user performing the augmentation procedure may use cutting device 108 to cut a slit 138 through the diseased leaflet as shown in FIG. 5A. In embodiments in which augmentation apparatus 100 does not include cutting device 108, the mitral valve leaflet may be cut using a separately introduced cutting device. Slit 138 is preferably cut in a direction substantially parallel to the free edge 20 of leaflet 14 and through tissue located in a belly 140 (e.g., between base 136 and free edge 20) of the leaflet. Slit 138 may be cut to a length that correlates to the desired length of the leaflet extension. That is, the user may create a short slit if a small extension is desired, or a longer slit if a longer extension is desired. After slit 138 has been cut, unsupported tissue located between the slit and free edge 20 may sag and cause the free edge to extend further from the base 136 of the leaflet, as shown in FIG. 5B.

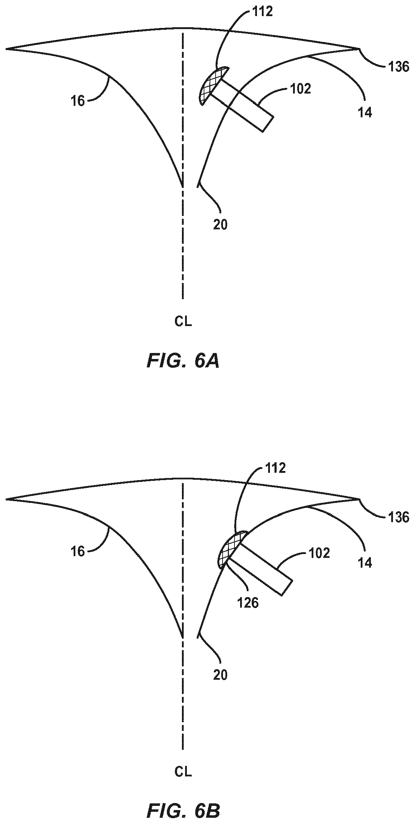

With plug 104 collapsed within the lumen 106 of delivery device 102, the user may insert a leading end of the delivery device through slit 138. As shown in FIG. 6A, the user may then utilize deployment device 134 to urge distal disk 112 from the lumen 106 of delivery device 102. Upon exiting lumen 106, distal disk 112 will transition to its expanded configuration.

After distal disk 112 has been deployed, the user may retract delivery device 102 until the inner surface 126 of distal disk 112 engages with the distal surface of posterior leaflet 14, as shown in FIG. 6B. With the inner surface 126 of distal disk 112 engaged with leaflet 14, the user may urge plug 104 completely from the lumen 106 of delivery device 102, causing proximal disk 110 to expand and the inner surface 122 of proximal disk 110 to engage the proximal surface of the leaflet, as shown in FIG. 6C. Expansion of proximal and distal disks 110, 112 creates a clamping force on opposing sides of leaflet 14 and secures plug 104 to the leaflet.

Full deployment of plug 104 also causes intermediate portion 114 to expand between the portions of tissue on opposite sides of slit 138, pushing these tissue portions away from one another. This expands the slit opening in the longitudinal direction of leaflet 14 and causes the free edge 20 of the leaflet to move further from the base 136 thereof. Leaflet 14 is thus lengthened to its augmented length L2, which is sufficient to restore proper coaptation of the mitral valve leaflets.

The user may then assess whether plug 104 has been properly positioned within leaflet 14. If the user determines that plug 104 has not been properly positioned, the user may retract deployment device 134, causing the plug to transition back to its collapsed configuration as it is withdrawn back into delivery device 102. The user may then redeploy plug 104 as described above until satisfied with its positioning. Once the user is satisfied with the positioning of plug 104 and leaflet 14 has been lengthened to its augmented length L2 such that proper coaptation has been restored, deployment device 134 may be rotated about its axis to unscrew the deployment device from clamp 130. Delivery device 102 may then be removed from the patient.

In certain circumstances, it may be desirable to augment the length of anterior leaflet 16 rather than posterior leaflet 14, or it may be desirable to augment the length of both the posterior leaflet and the anterior leaflet. In these situations, the length of either or both of the leaflets may be augmented as described above.

To summarize the foregoing, one aspect of the present disclosure is directed to a method of augmenting the length of a native leaflet. The method includes delivering a cutting tool to a surgical site adjacent the native leaflet; cutting a slit through the native leaflet; inserting a collapsible and expandable occlusion device into the slit, the occlusion device having a first disk, a second disk, and an intermediate portion coupling the first disk to the second disk; expanding the occlusion device; engaging a first surface of the first disk with a first surface of the native leaflet; and engaging a first surface of the second disk with a second surface of the native leaflet opposite the first surface; and/or
the native leaflet may be one of an anterior leaflet or a posterior leaflet of a native mitral valve; and/or
the slit may be cut through a belly of the native leaflet; and/or
the slit may be cut through the native leaflet in a direction substantially parallel to the free edge of the native leaflet; and/or
the step of expanding the occlusion device may cause the intermediate portion of the occlusion device to expand within the slit and move one section of the native leaflet away from another section of the naive leaflet; and/or
the cutting step may be performed using one of a blade, a saw, scissors, or a laser; and/or
the delivering step may include delivering the cutting tool in a delivery device using one of a transfemoral, transapical, or transseptal approach; and/or
the delivery device may include a flexible catheter; and/or
the inserting step may include pushing the occlusion device through a lumen of the delivery device using a deployment device; and/or
the method may further include retracting the deployment device relative to the delivery device to withdraw the occlusion device into the lumen of the delivery device; and/or
the method may further include rotating the deployment device relative to the occlusion device to uncouple the deployment device from the occlusion device; and/or
the occlusion device may be formed of a shape memory alloy that self-expand upon exiting the lumen of the delivery device; and/or
the expanding step may include expanding the intermediate portion of the occlusion device to a first dimension in a measurement direction orthogonal to a longitudinal axis of the occlusion device, and expending each of the first disk and the second disk to a dimension in the measurement direction greater than the first dimension.

Another aspect of the present disclosure is directed to an apparatus for augmenting the length of a native leaflet. The apparatus includes a delivery device having a lumen; and a collapsible and expandable plug having a first disk, a second disk, an intermediate portion coupling the first disk to the second disk, and a longitudinal axis extending through the first disk, the intermediate portion and the second disk, the first disk having a concave surface relative to a plane passing between the first disk and the second disk in a direction orthogonal to the longitudinal axis, the concave surface being adapted to engage one surface of the native leaflet, the second disk having a convex surface relative to the plane, the convex surface being adapted to engage a surface of the native leaflet opposite the one surface; and/or
the apparatus may further include a cutting device for cutting a slit through the native leaflet; and/or
the occlusion device may be formed from braided nitinol or a polylactide mesh; and/or
the braided nitinol or polylactide mesh may be covered by a fabric, a tissue or a polymeric material for substantially preventing blood flow through the occlusion device; and/or
the intermediate portion may have a dimension in a measurement direction orthogonal to a longitudinal axis of the occlusion device, and each of the first disk and the second disk have a dimension greater than the first dimension; and/or
the occlusion device may include a threaded clamp; and/or
the apparatus may further include a threaded deployment device configured to be removably coupled to the threaded clamp.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### INDUSTRIAL APPLICABILITY

The systems and methods described herein enable the repair of native heart valves that no longer function properly.

## Claims

1. An apparatus for augmenting the length of a native leaflet, comprising:
a delivery device having a lumen; and
a collapsible and expandable plug having a first disk, a second disk, an intermediate portion coupling the first disk to the second disk, and a longitudinal axis extending through the first disk, the intermediate portion and the second disk, the first disk having a concave surface relative to a plane passing between the first disk and the second disk in a direction orthogonal to the longitudinal axis, the concave surface being adapted to engage one surface of the native leaflet, and the second disk having a convex surface relative to the plane, the convex surface being adapted to engage a surface of the native leaflet opposite the one surface.

2. The apparatus of claim 1, wherein the delivery device is a flexible catheter.

3. The apparatus of claim 1, further comprising a cutting device for cutting a slit through the native leaflet.

4. The apparatus of claim 3, wherein the cutting device is one of a blade, a saw, scissors, or a laser.

5. The method of claim 1, wherein the occlusion device is formed of a shape memory alloy and self-expands upon exiting the lumen of the delivery device.

6. The apparatus of claim 5, wherein the occlusion device is formed from braided nitinol or polylactide mesh.

7. The apparatus of claim 6, wherein the braided nitinol or polylactide mesh is covered by a fabric, a tissue or a polymeric material for substantially preventing blood flow through the occlusion device.

8. The apparatus of claim 1, wherein the intermediate portion has a dimension in a measurement direction orthogonal to a longitudinal axis of the occlusion device, and each of the first disk and the second disk have a dimension greater than the first dimension.

9. The apparatus of claim 1, wherein the occlusion device includes a threaded clamp.

10. The apparatus of claim 9, further comprising a threaded deployment device configured to be removably coupled to the threaded clamp.
